# EUROPEAN PATENT APPLICATION

(11) **EP 2 357 216 A1**
(43) Date of publication of application: **17.08.2011**
(21) Application number: 09827550.6
(22) Date of filing: 17.11.2009
(51) Int. Cl.: C09K 8/34, C09K 8/035, C09K 8/04

(54) **BASE OIL FOR OIL DRILLING FLUID AND OIL DRILLING FLUID COMPOSITION**

(30) Priority: 18.11.2008 JP 2008294832
(71) Applicant: Idemitsu Kosan Co., Ltd., Chiyoda-ku Tokyo 100-8321 (JP)
(72) Inventor: TAKEUCHI, Kunio, Ichihara-shi Chiba 299-0193 (JP); FUJIKAWA, Shinjiro, Ichihara-shi Chiba 299-0193 (JP)
(74) Representative: Vossius & Partner
(86) International application number: PCT/JP2009/069488
(87) International publication number: WO 2010/058769

(57) **Abstract**

To provide an oil drilling fluid which is formed of an α-olefin oligomer produced from an α-olefin serving as a raw material in the presence of a metallocene catalyst, a base oil of the drilling fluid which has characteristics such as low toxicity and low aromatic content as well as high environmental suitability and which is suitable for oil drilling at low temperature.

## Description

### Technical Field

The present invention relates to a base oil for an oil drilling fluid base oil (hereinafter referred to as "oil drilling fluid base oil") and to an oil drilling fluid composition. More particularly, the invention relates to an oil drilling fluid base oil and an oil drilling fluid composition which are suitable for offshore oil drilling.

### Background Art

In recent oil-field development, wells are drilled through the rotary drilling technique generally employing a drilling rig and a rotating bit. In the drilling operation, a fluid so-called "mud" is employed for transferring and removing cuttings, adjusting the pressure in the well, preventing breaking of the well, and cooling and lubricating the bit and other elements. Therefore, in order to attain appropriate specific weight, viscosity, lubricity, etc., a variety of ingredients are added to the mud.

In recent years, new oil fields such as those in cold regions (e.g., Alaska) and deep-sea oil fields are targeted. However, since the temperatures in such cold regions and deep sea are very low, mud exhibits different characteristics due to low temperature. Thus, there is demand for a mud which is also suitable for drilling operation at such a site. From another aspect, impact on the environment must be considered in recent development of oil fields. In particular, development of offshore oil fields must be carried out while reducing negative effects on marine organisms and the natural environment.

Hitherto, mineral oil is employed as a lube oil for offshore oil drilling or a like purpose and is incorporated into a mud. However, in order to meet the aforementioned demand in relation to drilling at low temperature and the environment, mineral oil has been shifted to synthetic oil in recent years.

Poly-α-olefin is a generally known synthetic oil for use as a lube oil. For example, Patent Document 1 discloses a composition containing a α-olefin dimer produced in the presence of BF₃. However, the composition has high viscosity at low temperature, which is not suited for oil drilling at low temperature. Patent Document 2 discloses a drilling oil containing poly-α-olefin. The characteristics of the disclosed oil are not necessarily sufficient, when recent rigorous environmental regulations with respect to the environment are taken into consideration.

### Prior Art Document

### Patent Documents

Patent Document 1: US Patent No. 5171905
Patent Document 2: US Patent No. 5045219

### Summary of the Invention

### Problems to be Solved by the Invention

The present invention has been conceived under such circumstances. Thus, an object of the present invention is to provide a drilling fluid base oil which has characteristics such as low toxicity and low aromatic content as well as high environmental suitability and which is suitable for oil drilling at low temperature.

### Means for Solving the Problems

The present inventors have conducted extensive studies, and have found that the aforementioned object can be attained by use of an α-olefin oligomer which is produced in the presence of a metallocene catalyst. The present invention has been accomplished on the basis of this finding. Accordingly, the present invention is directed to the following.
1. An oil drilling fluid base oil which is formed of an α-olefin oligomer produced from an α-olefin serving as a raw material in the presence of a metallocene catalyst.
2. The oil drilling fluid base oil according to 1 above, wherein the α-olefin oligomer has a kinematic viscosity as measured at 0°C of 18 mm²/s or less.
3. The oil drilling fluid base oil according to 1 or 2 above, wherein the α-olefin oligomer has a kinematic viscosity as measured at 100°C of 2 mm²/s or less.
4. The oil drilling fluid base oil according to any of 1 to 3 above, wherein the α-olefin oligomer has a kinematic viscosity as measured at 40°C of 7 mm²/s or less.
5. The oil drilling fluid base oil according to any of 1 to 4 above, wherein the α-olefin oligomers is an average C16 to C22 α-olefin oligomer.
6. The oil drilling fluid base oil according to any of 1 to 5 above, wherein the α-olefin is a C4 to C12 α-olefin.
7. The oil drilling fluid base oil according to any of 1, to 6 above, wherein the α-olefin is a C8 to C10 α-olefin.
8. The oil drilling fluid base oil according to any of 1 to 7 above, wherein the α-olefin oligomer is 1-octene and/or 1-decene.
9. The oil drilling fluid base oil according to any of 1 to 8 above, wherein the α-olefin oligomer has a dimer content of 80 mass% or more with respect to the total amount of the α-olefin oligomer.
10. The oil drilling fluid base oil according to any of 1 to 9 above, wherein the α-olefin oligomer has a dimer content of 90 mass% or more with respect to the total amount of the α-olefin oligomer.
11. The oil drilling fluid base oil according to any of 1 to 10 above, wherein the metallocene catalyst comprises a metallocene compound represented by formula (I):

   (RC₅H₄)₂MX₂ (I)

   (wherein R represents a hydrogen atom or a C1 to C10 hydrocarbyl group; M represents a group 4 transition metal element in the periodic table; and X represents a covalent-bonding or ionically bonding ligand) and a methylaluminoxane.
12. The oil drilling fluid composition comprising an oil drilling fluid base oil as recited in any of 1 to 11 above.

### Effects of the Invention

The present invention can provide a drilling fluid base oil which has characteristics such as low toxicity and low aromatic content as well as high environmental suitability and which maintains low viscosity even at low temperature. By use of the oil drilling fluid base oil, drilling in cold regions and drilling of deep-sea oil fields can be effectively performed.

### Modes for Carrying Out the Invention

The oil drilling fluid base oil of the present invention is formed of an α-olefin oligomer produced from an α-olefin serving as a raw material in the presence of a metallocene catalyst. As used herein, the term "oligomer" refers to a polymer which is produced through polymerization of a monomer or to a composition of the polymer. The oligomer may be a single specific polymer or a mixture of two or more species (e.g., dimer and trimer).

The α-olefin oligomer employed in the present invention preferably has a kinematic viscosity as measured at 0°C of 18 mm²/s or less. When the kinematic viscosity as measured at 0°C is 18 mm²/s or less, drilling operation can be effectively performed even at low temperature. From this viewpoint, the kinematic viscosity as measured at 0°C is more preferably 5.0 to 17.0 mm²/s, particularly preferably 5.0 to 15.0 mm²/s. An α-olefin oligomer having a kinematic viscosity as measured at 0°C of 18.0 mm²/s or less can be produced through, for example, elevating the dimer content. The dimer content is generally 80 mass% or more with respect to the total amount of the α-olefin oligomer, preferably 90 mass% or more.

The α-olefin oligomer employed in the present invention preferably has a kinematic viscosity as measured at 100°C of 2 mm²/s or less, more preferably 1.0 to 1.8 mm²/s. An α-olefin oligomer having a kinematic viscosity as measured at 100°C of 2 mm²/s or less can be produced through, for example, employing a C≤10 α-olefin as a monomer.

The α-olefin oligomer employed in the present invention preferably has a kinematic viscosity as measured at 40°C of 7 mm²/s or less, more preferably 2.0 to 7.0 mm²/s. An α-olefin oligomer having a kinematic viscosity as measured at 40°C of 7 mm²/s or less can be produced through, for example, employing a C≤8 α-olefin as a monomer.

The α-olefin oligomer employed in the present invention preferably has, on average, 16 to 22 carbon atoms, more preferably 16 to 20, particularly preferably 16 to 18, most preferably 16 to 17.

The α-olefin employed as a raw material of the α-olefin oligomer is generally a C4 to C12 linear α-olefin. Specific examples of the α-olefin include 1-butene, 1-octene, 1-hexene, 1-haptene, 1-octene, 1-nonene, 1-decene, and 1-dodecene. When a C4 to C12 α-olefin is employed, α-olefin oligomers satisfying the aforementioned kinematic viscosity conditions can be readily produced. From this viewpoint, a C8 to C10 α-olefin is preferably employed, and 1-octene or 1-decene is more preferred. In the present invention, α-olefins may be used singly or in combination of two or more species.

The α-olefin oligomer employed in the present invention preferably has a dimer content of 80 mass% or more with respect to the total amount of the α-olefin oligomer, more preferably 90 mass% or more. When the dimer content is 80 mass% or more, excellent viscosity characteristics thereof can be attained, and α-olefin oligomers satisfying the aforementioned kinematic viscosity conditions at 0°C can be readily produced. In a dimer having a vinylidene group (hereinafter may be referred to as vinylidene-containing form), the dimer content with respect to the total amount is preferably 80 mass% or more, more preferably 90 mass% or more.

The α-olefin oligomer employed in the present invention preferably has a pour point of -5°C or lower, more preferably -10°C or lower. When the pour point is -5°C or lower, drilling operation can be effectively performed even at low temperature.

The oil drilling fluid base oil according to the present invention is formed of the aforementioned α-olefin oligomer. The α-olefin oligomer has characteristics such as low toxicity and low aromatic content as well as high environmental suitability and excellent viscosity characteristics at low temperature. Thus, the oil drilling fluid base oil according to the present invention is preferably employed in oil drilling at low temperature, in particularly in offshore oil drilling.
The oil drilling fluid composition the present invention is produced by mixing the oil drilling fluid base oil with an additive. The oil drilling fluid composition also has high environmental suitability and excellent viscosity characteristics at low temperature. Thus, the composition is preferably employed in oil drilling at low temperature, in particularly in offshore oil drilling.
No particular limitation is imposed on the mode of use of the oil drilling fluid base oil or the oil drilling fluid composition. The base oil or composition may be directly applied to a machine for lubrication or may be employed as a mud component.

The metallocene catalyst serving as the catalyst employed in production of the aforementioned α-olefin oligomer is, for example, a combination of a metallocene compound and a co-catalyst. The metallocene compound is preferably a metallocene compound represented by formula (I):

(RC₅H₄)₂MX₂ (I)

(wherein R represents a hydrogen atom or a C1 to C10 hydrocarbyl group; M represents a group 4 transition metal element in the periodic table; and X represents a covalent-bonding or ionically bonding ligand).

In formula (I), R is preferably a hydrogen atom or a C1 to C4 hydrocarbyl group. Specific examples of M include titanium, zirconium, and hafnium. Among them, zirconium is preferred. Specific examples of X include a hydrogen atom, a halogen atom, a C1 to C20 (preferably C1 to C10) hydrocarbyl group, a C1 to C20 (preferably C1 to C10) alkoxy group, an amino group, a C1 to C20 (preferably C1 to C12) phosphorus-containing hydrocarbyl group (e.g., a diphenylphosphine group), a C1 to C20 (preferably C1 to C12) silicon-containing hydrocarbyl group (e.g., a trimethylsilyl group), a C1 to C20 (preferably C1 to C12) hydrocarbyl group; and a halo-containing boron compound (e.g., B(C₆H₅)₄ or BF₄). Of these,
a hydrogen atom, a halogen atom, a hydrocarbyl group, and an alkoxy group are preferred.

Specific examples of the metallocene compound represented by formula (I) include bis(cyclopentadienyl)zirconium dichloride, bis(methylcyclopentadienyl)zirconium dichloride, bis(ethylcyclopentadienyl)zirconium dichloride, bis (iso-propylcyclopentadienyl)zirconium dichloride, bis(n-propylcyclopentadienyl)zirconium dichloride, bis(n-butylcyclopentadienyl)zirconium dichloride, bis(t-butylcyclopentadienyl)zirconium dichloride, bis(hexylcyclopentadienyl)zirconium dichloride, bis(trimethylsilylcyclopentadienyl)zirconium dichloride, bis(trimethylsilylmethylcyclopentadienyl)zirconium dichloride, bis(cyclopentadienyl)zirconium chlorohydride, bis(cyclopentadienyl)methylzirconium chloride, bis(cyclopentadienyl)ethylzirconium chloride, bis(cyclopentadienyl)methoxyzirconium chloride, bis (cyclopentadienyl)phenylzirconium chloride, bis(cyclopentadienyl)dimethylzirconium, bis(cyclopentadienyl)diphenylzirconium, bis(cyclopentadienyl)dineopentylzirconium, bis(cyclopentadienyl)dihydrozirconium, and bis(cyclopentadienyl)dimethoxyzirconium. Specific examples thereof further include the aforementioned compounds in which the chlorine atom is substituted by a bromine atom, an iodine atom, a hydrogen atom, a methyl group, a phenyl group, etc., and the aforementioned compounds in which zirconium (the center metal) is substituted by titanium or hafnium.

The aforementioned co-catalyst is preferably a methylaluminoxane. No particular limitation is imposed on the methylaluminoxane, and conventionally known methylaluminoxane forms may be employed. Examples include chain or cyclic methylaluminoxanes represented by formula (II) or (III):

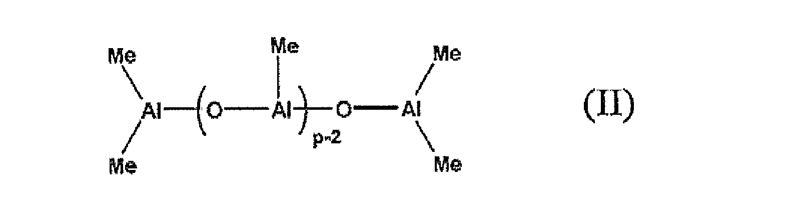

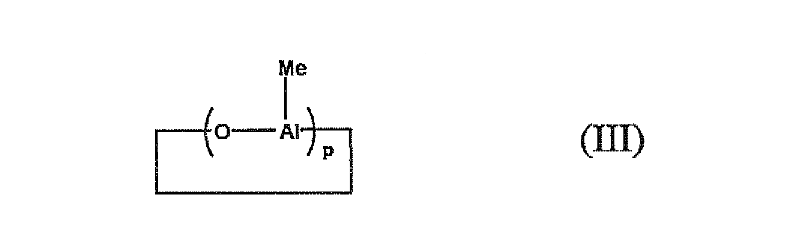

(wherein p represents a polymerization degree which is generally 3 to 50, preferably 7 to 40).

No particular limitation is imposed on the method for producing the methylaluminoxane, and raw materials may be reacted in accordance with a known method, for example, contact between methylaluminum and a condensing agent such as water.

The ratio of methylaluminoxane to metallocene compound, methylaluminoxane/metallocene compound (ratio by mole), is generally 1 to 1,000, preferably 1 to 100, more preferably 1 to 30. When the mole ratio is 1 or more, sufficient catalytic activity can be attained, whereas when the ratio is 1,000 or less, formation of higher polymeric forms can be prevented.
The ratio of α-olefin to metallocene compound represented by formula (I), metallocene compound (mmol)/α-olefin (L), is generally 0.01 to 2, preferably 0.02 to 1, more preferably 0.05 to 0.5. When the ratio is 0.01 or more, sufficient catalytic activity can be attained, whereas when the ratio is 2 or less, catalyst residues can be readily removed.

No particular limitation is imposed on the reaction mode of oligomerization, and the reaction may be performed in the presence or absence of solvent. Examples of the reaction solvent which is optionally employed include aromatic hydrocarbons such as benzene, toluene, xylene, and ethylbenzene; alicyclic hydrocarbons such as cyclopentane, cyclohexane, and methylcyclohexane; aliphatic hydrocarbons such as pentane, hexane, heptane, and octane; and halohydrocarbons such as chloroform and dichloromethane. The oligomerization temperature is generally 0 to 100°C, preferably 20 to 80°c, more preferably 30 to 70°C. Under the temperature conditions, sufficient catalytic activity can be attained.

The oligomerization reaction may be performed in the presence of an aluminum compound such as diethylaluminum chloride. By virtue of the presence of the aluminum compound, the polymerization activity can be enhanced, and the polymerization degree of the oligomer can be controlled. Also, the oligomerization reaction may be performed in the presence of hydrogen. The amount of hydrogen employed in the reaction is generally 0 to 50 kPa, preferably 0 to 30 kPa, more preferably 0 to 10 kPa. When the amount of hydrogen falls within the range, formation of the saturated form of the raw material α-olefin is prevented, thereby attaining enhanced yield of the target α-olefin oligomer.

The α-olefin oligomer produced through the above procedure may be subjected to hydrogenation. Through hydrogenation, stability against heat and oxidation can be enhanced. The hydrogenation temperature is generally 50 to 300°C, preferably 60 to 250°C, more preferably 70 to 200°C, and the hydrogen pressure is generally 0.1 to 10 MPa, preferably 0.5 to 2 MPa, more preferably 0.7 to 1.5 MPa. In hydrogenation, conventional hydrogenation catalyst containing Pd, Ni, etc. may be employed. Through distillation of an α,-olefin oligomer or a hydrogenation product thereof, a fraction having a target kinematic viscosity can be yielded. The distillation temperature is generally 100 to 300°C, preferably 120 to 280°C, more preferably 140 to 260°C, and the distillation pressure is generally 0.1 to 15 Pa, preferably 0.4 to 7 Pa, more preferably 0.6 to 4 Pa. Examples

The present invention will next be described in more detail by way of examples, which should not be construed as limiting the invention thereto.

The yields of oligomers, the proportions of formed oligomers, and the vinylidene-group-containing form content of a dimer were determined through gas chromatography- The kinematic viscosity was measured at 0°C, 40°C, and 100°C in accordance with JIS K2283, The pour point was measured in accordance with JIS K2269. The flash point was measured in accordance with JIS K2265 (Cleveland open-cup flash point test).

The details of gas chromatography conditions are as follows.

### [Yields of oligomers and proportions thereof]

Column: HT-STMDST (5 m × 0.53 mm × 0.17 µm)

Carrier flow: 40 cm/sec

Injection mode: cool-on-column injection

Injection temperature and detection temperature: 440°C Column temperature: 50°C (retention: 0.1 min), elevation at 20°C/min, 430°C (retention: 15 min)

INJ amount: 0.5 µL

Sample concentration: 1 wt.% toluene solution (containing hexadecane internal standard (1 wt.%))

[Vinylidene-group-containing form content of dimer]

Column: ultra 2 (25 m × 0.20 mm × 0.33 µm)

Carrier flow: 41.7 cm/sec

Injection mode: split ratio of 30

Injection temperature and detection temperature: 300°C Column temperature: 100°C (retention: 1 min), elevation at 10°C/min, 300°C (retention: 20 min)

INJ amount: 1 µL

Sample concentration: 1 wt.% toluene solution

### Example 1

To an autoclave (capacity: 2 L) made of stainless steel having a nitrogen atmosphere, 1-decene (1 L) which had been degassed and dehydrated through nitrogen bubbling was added. Subsequently, a solution (1.0 mol/L, 5 mL) of methylaluminoxane in toluene and a solution (1.0 mol/L, 1 mL) of diethylaluminum chloride in toluene were added to the autoclave- The mixture was heated to 50°C and stirred. A solution (20 mmol/L, 25 mL) of bis(cyclopentadienyl)zirconium dichloride in toluene was further added to the autoclave, and the resultant mixture was allowed to react at 50°C for 20 hours. The reaction was terminated with 1% aqueous sodium hydroxide (250 mL). The reaction mixture was washed twice with deionized water (50 mL), to thereby decompose and remove the catalyst components. Through gas chromatographic analysis of the thus-obtained solution, the yield of oligomers was 87%, and the oligomer product was found to include dimer (91.5%), trimer (6.1%), tetramer (1.5%), pentamer (0.5%), and hexamer and higher oligomers (0.4%). The oligomer solution was distilled at 1.33 Pa and 200°C by means of a simple distillator. The distillate was found to be an oligomer mixture containing dimer (99.9%) and trimer (0.1%). The dimer was found to contain vinylidene-group-containing form in an amount of 98.2%. Table 1 shows the physical properties of the oligomer product.

### Example 2

To an autoclave (capacity: 2 L) made of stainless steel having a nitrogen atmosphere, 1-octene (1 L) which had been degassed and dehydrated through nitrogen bubbling was added. Subsequently, a solution (1.0 mol/L, 5 mL) of methylaluminoxane in toluene and a solution (1.0 mol/L, 1 mL) of diethylaluminum chloride in toluene were added to the autoclave. The mixture was heated to 50°C and stirred. A solution (20 mmol/L, 25 mL) of bis(cyclopentadienyl)zirconium dichloride in toluene was further added to the autoclave, and the resultant mixture was allowed to react at 50°C for 20 hours. The reaction was terminated with 1% aqueous sodium hydroxide (250 mL). The reaction mixture was washed twice with deionized water (50 mL), to thereby decompose and remove the catalyst components. Through gas chromatographic analysis of the thus-obtained solution, the yield of oligomers was 88%, and the oligomer product was found to include dimer (92.1%), trimer (5-8%), tetramer (1.3%), pentamer (0.5%), and hexamer and higher oligomers (0.3%). The dimer was found to contain vinylidene-group-containing form in an amount of 99.0%. Table 1 shows the physical properties of the oligomer product.

### Example 3

To an autoclave (capacity: 5 L) made of stainless steel having a nitrogen atmosphere, 1-decene (2.5 L) which had been degassed and dehydrated through nitrogen bubbling was added. Subsequently, a solution (1.0 mol/L, 12 mL) of methylaluminoxane in toluene was added to the autoclave. The mixture was heated to 50°C and stirred. A solution (40 mmol/L, 10 mL) of bis(cyclopentadienyl)zirconium dichloride in toluene was further added to the autoclave, and the resultant mixture was allowed to react at 50°C for 7 hours under stirring, while hydrogen (5 kPa) was continuously fed to the autoclave. The reaction was terminated with 1% aqueous sodium hydroxide (500 mL). The reaction mixture was washed twice with deionized water (100 mL), to thereby decompose and remove the catalyst components. Through gas chromatographic analysis of the thus-obtained solution, the yield of oligomers was 94%, and the oligomer product was found to include dimer (42%), trimer (11%), tetramer (7%), pentamer (5%), and hexamer and higher oligomers (35%). The oligomer solution was distilled at 1.33 Pa and 200°C by means of a simple distillator. The distillate was found to be an oligomer mixture containing dimer (99.4%) and trimer (0.6%). The dimer was found to contain vinylidene-group-containing form in an amount of 95-5%- Table 1 shows the physical properties of the oligomer product.

### Example 4

To an autoclave (capacity: 5 L) made of stainless steel having a nitrogen atmosphere, 1-decene (2.5 L) which had been degassed and dehydrated through nitrogen bubbling was added. Subsequently, a solution (1.0 mol/L, 6 mL) of methylaluminoxane in toluene was added to the autoclave. The mixture was heated to 50°C and stirred. A solution (25 mmol/L, 10 mL) of bis(t-butylcyclopentadienyl)zirconium dichloride in toluene was further added to the autoclave, and the resultant mixture was allowed to react at 50°C for 5 hours under stirring, while hydrogen (2.5 kPa) was continuously fed to the autoclave. The reaction was terminated with 1% aqueous sodium hydroxide (250 mL). The reaction mixture was washed twice with deionized water (50 mL), to thereby decompose and remove the catalyst components. Through gas chromatographic analysis of the thus-obtained solution, the yield of oligomers was 92%, and the oligomer product was found to include dimer (42%), trimer (24%), tetramer (12%), pentamer (7%), and hexamer and higher oligomers (15%). The oligomer solution was distilled at 1.33 Pa and 200°C by means of a simple distillator. The distillate was found to be an oligomer mixture containing dimer (99.6%) and trimer (0.4%). The dimer was found to contain vinylidene-group-containing form in an amount of 93.5%. Table 1 shows the physical properties of the oligomer product.

### Comparative Example 1

Table 1 also shows the physical properties of a commercial α-olefin oligomer, which was produced in the presence of a BF₃ catalyst. As is clear from Table 1, this oligomer has a high kinematic viscosity as measured at 0°C, which is not suited for drilling at low temperature.

### Comparative Example 2

A stainless steel reaction column (diameter: 12 mm, length: 1.1 m, inner diameter: 10 mm) was filled with HMFI-90 (proton-type MFI zeolite catalyst, product of Sud Chemie) (50 mL). While nitrogen gas was fed to the reaction column at 100 mL/min, the catalyst was preliminarily conditioned at 200°C for 24 hours.

The reaction column was cooled to 100°C, and a mixture of C16 α-olefin (70 mass%) and C18 α-olefin (30 mass%) was upwardly fed to the column at 100 mL/hour. The fed α-olefin mixture had an oxo compound content of 12 ppm by mass (as reduced to oxygen) and a water content of 5 ppm by mass. The raw material C16 α-olefin had a linear ratio (linear olefin content) of 95%, and the C18 α-olefin had a linear ratio 90%. The reaction temperature was gradually elevated from a start of feeding the α-olefin mixture and adjusted to 160°C at hour 350. At this point in time, the raw material α-olefines exhibited the following percent double bond isomerization values: 96% (C16 α-olefin) and 95% (C18 α-olefin). The thus-formed internal olefin composition was found to include C16 olefin (60 mass%) and C18 olefin (40 mass%) and to have linear ratio of 95% (C46 olefin) and 90% (C18 α-olefin). The internal olefin composition was found to have a raw material α-olefin of 4.4 mass%, a branched olefin (skeleton isomerization product) content of 7.0 mass%, and a heavy component (dimerization product of α-olefin and other products) content of 1.7 mass%. Table 1 shows the kinematic viscosity, pour point, and flash point of the produced internal olefin composition. As is clear from table 1, the pour point is excessively high, which is not suited for drilling at low temperature.

[Table 1]

**Table 1**

| | | Examples | | | | Comp. Exs. | |
|---|---|---|---|---|---|---|---|
| | | 1 | 2 | 3 | 4 | 1 | 2 |
| Kinematic viscosity (mm²/s) | 0°C | 14.4 | 7.1 | 15.0 | 14.7 | 19.0 | 7.9 |
| | 40°C | 4.5 | 2.7 | 4.5 | 4.5 | 5.0 | 3.0 |
| | 100°C | 1.7 | 1.1 | 1.7 | 1.7 | 1.7 | 1.3 |
| Flash point (°C) | | 176 | 136 | 174 | 176 | 157 | 142 |
| Pour point (°C) | | -15.0 | -42.5 | -15.0 | -15.0 | -66.0 | -9.0 |

### [Environmental suitability]

The α-olefin oligomers produced in the Examples and Comparative Examples were subjected to the following tests. Table 2 shows the results.
Fish acute toxicity test: Performed in accordance with ASTM E1367-99 (Standard Guide for Conducting 10-day Static Sediment Toxicity Tests with Marine and Estuarine Amphipoda). The results were evaluated on the basis of EPA (Environmental Protection Agency) regulation (GMG290000: LC₅₀ test sample/LC₅₀ standard sample≤1).
Lower aromatic test: performed in accordance with PAH (polyaromatic-hydrocarbon) test EPA1654A.

[Table 2]

**Table 2**

| | Examples | | | | Comp. Exs. | |
|---|---|---|---|---|---|---|
| | 1 | 2 | 3 | 4 | 1 | 2 |
| Fish acute toxicity test | 0.5 | 0.9 | 0.5 | 0.5 | 0.3 | 0.7 |
| Lower aromatic hydrocarbon test | ND | ND | ND | ND | ND | ND |

As is clear from Table 2, the α-olefin oligomers of Comparative Examples exhibited unsatisfactory performance for use as a lube oil for oil drilling, although satisfactory environmental suitability was attained. In contrast, α-olefin oligomers of Examples sufficiently satisfied the EPA regulation established for offshore oil drilling (environmental suitability) and exhibited excellent performance for use as a lube oil for oil drilling.

### Industrial Applicability

The present invention is enables provision of a drilling fluid base oil which has characteristics such as low toxicity and low aromatic content as well as high environmental suitability and which has low viscosity at low temperature. Through employment of the oil drilling fluid base oil, drilling in cold regions and drilling of deep-sea oil fields can be effectively performed, wear of drilling machines can be reduced, and the service life of a drilling machine can be prolonged.

## Claims

1. An oil drilling fluid base oil which is formed of an α-olefin oligomer produced from an α-olefin serving as a raw material in the presence of a metallocene catalyst.

2. The oil drilling fluid base oil according to claim 1, wherein the α-olefin oligomer has a kinematic viscosity as measured at 0°C of 18 mm²/s or less.

3. The oil drilling fluid base oil according to claim 1 or 2, wherein the α-olefin oligomer has a kinematic viscosity as measured at 100°C of 2 mm²/s or less.

4. The oil drilling fluid base oil according to any of claims 1 to 3, wherein the α-olefin oligomer has a kinematic viscosity as measured at 40°C of 7 mm²/s or less.

5. The oil drilling fluid base oil according to any of claims 1 to 4, wherein the α-olefin oligomer is an average C16 to C22 α-olefin oligomer.

6. The oil drilling fluid base oil according to any of claims 1 to 5, wherein the α-olefin is a C4 to C12 α-olefin.

7. The oil drilling fluid base oil according to any of claims 1 to 6, wherein the α-olefin is a C8 to C10 α-olefin.

8. The oil drilling fluid base oil according to any of claims 1 to 7, wherein the α-olefin is 1-octene and/or 1-decene.

9. The oil drilling fluid base oil according to any of claims 1 to 8, wherein the α-olefin oligomer has a dimer content of 80 mass% or more with respect to the total amount of the α-olefin oligomer.

10. The oil drilling fluid base oil according to any of claims 1 to 9, wherein the α-olefin oligomer has a dimer content of 90 mass% or more with respect to the total amount of the α-olefin oligomer.

11. The oil drilling fluid base oil according to any of claims 1 to 10, wherein the metallocene catalyst comprises a metallocene compound represented by formula (I):
(RC₅H₄)₂MX₂ (I)
(wherein R represents a hydrogen atom or a C1 to C10 hydrocarbyl group; M represents a group 4 transition metal element in the periodic table; and X represents a covalent-bonding or ionically bonding ligand) and a methylaluminoxane-12. The oil drilling fluid composition comprising an oil drilling fluid base oil as recited in any of claims 1 to 11.
